(19) **Europäisches Patentamt**
**European Patent Office**
**Office européen des brevets**

(11) **EP 4 374 158 B1**

(12) **FASCICULE DE BREVET EUROPEEN**

(45) Date de publication et mention
de la délivrance du brevet:
**05.11.2025 Bulletin 2025/45**

(51) Classification Internationale des Brevets (IPC):
*G01N 21/75* (2006.01)   *G01N 21/64* (2006.01)
*G01N 21/27* (2006.01)   *G01N 21/76* (2006.01)
*G01N 21/77* (2006.01)

(21) Numéro de dépôt: **22754116.6**

(22) Date de dépôt: **19.07.2022**

(52) Classification Coopérative des Brevets (CPC):
**G01N 21/272; G01N 21/6408; G01N 21/6452;**
**G01N 21/6456; G01N 21/75;** G01N 21/76;
G01N 2021/752; G01N 2021/755; G01N 2021/7786

(86) Numéro de dépôt international:
**PCT/FR2022/051428**

(87) Numéro de publication internationale:
**WO 2023/002114 (26.01.2023 Gazette 2023/04)**

(54) **PROCÉDÉ DE QUANTIFICATION D'ENDOTOXINES D'UN ÉCHANTILLON BIOLOGIQUE**

VERFAHREN ZUR QUANTIFIZIERUNG VON ENDOTOXINEN IN EINER BIOLOGISCHEN PROBE

METHOD FOR QUANTIFYING ENDOTOXINS IN A BIOLOGICAL SAMPLE

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorité: **19.07.2021 FR 2107772**

(43) Date de publication de la demande:
**29.05.2024 Bulletin 2024/22**

(73) Titulaire: **BIOMERIEUX**
**69280 Marcy-L'Etoile (FR)**

(72) Inventeurs:
• **GOUJARD, Matthieu**
**38000 GRENOBLE (FR)**
• **BROYER, Patrick**
**38500 SAINT-CASSIEN (FR)**
• **PINSTON, Frédéric**
**38000 GRENOBLE (FR)**

(74) Mandataire: **bioMérieux PI Groupement**
**mandataires**
**bioMérieux**
**69280 Marcy l'Etoile (FR)**

(56) Documents cités:
**WO-A1-2012/118226    WO-A1-2018/226824**
**WO-A1-2020/185646    US-A1- 2009 269 858**
**US-A1- 2012 003 745    US-A1- 2020 309 695**

• **HARUKI OISHI ET AL: "DEVELOPMENT OF A**
**MULTICHANNEL TURBIDIMETRIC-KINETIC**
**PHOTOMETER FOR THE QUANTITATIVE**
**DETERMINATION OF ENDOTOXIN", APPLIED**
**SPECTROSCOPY, THE SOCIETY FOR APPLIED**
**SPECTROSCOPY. BALTIMORE, US, vol. 43, no.**
**5, July 1989 (1989-07-01), pages 821 - 826,**
**XP000029360, ISSN: 0003-7028, DOI: 10.1366/**
**0003702894202319**

EP 4 374 158 B1

## Description

## Domaine technique

La présente invention se rapporte au domaine de l'analyse d'échantillons biologiques, et plus précisément concerne un procédé de quantification d'endotoxines d'un échantillon biologique au moyen d'un instrument de mesure.

## Arrière-plan technologique

Les endotoxines sont des toxines situées dans la membrane externe de certaines bactéries à Gram négatif, de nature lipopolysaccharidique (LPS) et thermostables. Ce sont des substances pyrogènes, c'est-à-dire pouvant provoquer de fortes fièvres. Les normes de la pharmacopée imposent l'absence de ce type de substances dans les produits pharmaceutiques qui entrent en contact avec la circulation sanguine ou le système nerveux central, comme les médicaments injectables ou les dispositifs médicaux. Il est également recommandé de quantifier les endotoxines dans les matières premières telles que l'eau ou les en-cours de production.

La détection des endotoxines repose aujourd'hui essentiellement sur l'utilisation de réactifs élaborés à partir d'une fraction purifiée de sang de limules, une famille de crabes en voie de disparition en Asie et protégés aux États Unis et dont le sang a la propriété de coaguler en présence d'infimes quantités d'endotoxines bactériennes. Il a été développé une nouvelle approche basée sur des protéines recombinantes du Facteur C de limule (rFC), permettant de s'affranchir de l'utilisation de sang de limules et de détecter ainsi la présence d'endotoxines.

Selon un procédé de détection typique d'endotoxine, plusieurs solutions d'endotoxines à des concentrations standardisées sont préparées, par exemple à 50 UE/mL, 5 EU/mL, 0,5 EU/mL. UE correspond à l'anglais "Endotoxin Unit" pour Unité Endotoxine, et est une mesure de l'activité des endotoxines, équivalente à l'unité internationale, ou IU pour "International Unit".

Cette approche repose encore sur la détermination quantitative in vitro de l'endotoxine dans des échantillons pharmaceutiques, biologiques et environnementaux. Ces tests sont exigeants et requièrent beaucoup d'étapes de manipulation par l'opérateur, ils nécessitent la préparation de dilutions standard et de contrôles internes. Ces étapes manuelles de préparation sont longues et peuvent entraîner des résultats variables voire invalides.

En outre, les procédés actuels se basent uniquement sur les résultats obtenus à l'issue d'une durée de mesure fixe, par exemple de 90 minutes, pendant laquelle l'échantillon biologique à analyser est placé à une température donnée, typiquement d'environ 37°C. Cette durée de mesure fixe est commune à toutes les mesures, et a été préalablement choisie afin d'être suffisamment longue pour permettre l'achèvement complet des diverses réactions susceptibles de se produire avec différentes dynamiques. Par conséquent, pour la plupart des mesures, cette durée de mesure est beaucoup plus longue que nécessaire, et malgré cela ce temps de mesure peut encore être trop court pour quelques mesures dans des cas particuliers. En outre, en cas de défaut de configuration de la mesure, par exemple une erreur de manipulation, un éventuel problème ne pourra être détecté qu'à la fin de la durée de mesure, lorsqu'il sera tenté d'exploiter les résultats erronés. Le document WO 2020/185646 A1 divulgue un procédé de quantification d'endotoxines d'un échantillon biologique utilisant l'évolution temporelle d'un signal de fluorescence associé à une réaction entre des réactifs et la présence d'endotoxines dans l'échantillon.

## Présentation de l'invention

L'invention vise donc à permettre de suivre l'évolution temporelle d'une concentration d'endotoxines dans l'échantillon biologique au cours de la durée de mesure, de façon fiable à chaque instant de mesure.

A cet effet, l'invention propose un procédé de quantification d'endotoxines d'un échantillon biologique par un instrument d'analyse comprenant un imageur définissant un champ de vue, un support d'analyse étant introduit dans le champ de vue de l'instrument d'analyse, ledit support d'analyse comprenant au moins une chambre d'analyse configurée pour recevoir ledit échantillon biologique et une pluralité de chambres de référence configurées pour recevoir un liquide de référence, le procédé comprenant d'abord une mise en place de l'échantillon biologique dans ladite chambre d'analyse et du liquide de référence dans la pluralité de chambres de référence, des chambres de référence étant pourvues de réactifs de référence et de différentes concentrations d'endotoxines, lesdits réactifs de référence étant aptes à causer une réaction de luminescence en présence du liquide de référence en fonction de la concentration d'endotoxines dans la chambre de référence correspondante, la chambre d'analyse étant pourvue de réactifs d'analyse aptes à causer une réaction de luminescence en présence d'endotoxines de l'échantillon biologique,

le procédé comprenant, pour chaque instant de mesure d'une durée de mesure, l'acquisition audit instant de mesure d'une image du support d'analyse et la détermination, à partir de ladite image du support d'analyse, d'une valeur d'intensité lumineuse de chambre d'analyse pour ledit instant de mesure,
le procédé comprenant en outre, pour une pluralité d'instants de mesure :

- une détermination à partir de l'image du support d'analyse, de valeurs d'intensité lumineuse de chambres de référence pour ledit instant de mesure,

- une détermination, à partir des valeurs d'intensité lumineuse de chambres de référence pour ledit instant de mesure, d'une relation d'étalonnage liant la valeur d'intensité lumineuse et concentration d'endotoxines à cet instant de mesure ;

le procédé comprenant également, pour une pluralité d'instants de mesure:

- une détermination d'au moins une mesure de concentration d'endotoxines dans l'échantillon biologique à cet instant de mesure à partir d'une relation d'étalonnage pour ledit instant de mesure et de la valeur d'intensité de chambre d'analyse audit instant de mesure ; et
- une détermination d'une évolution temporelle de la mesure de concentration d'endotoxines dans l'échantillon biologique au cours de la durée de mesure à partir de mesures de concentration d'endotoxines pour plusieurs instants de mesure.

L'invention est avantageusement complétée par les différentes caractéristiques suivantes prises seules ou selon leurs différentes combinaisons possibles :

- le procédé comprend ensuite une mise en œuvre d'une action fonction de l'évolution temporelle de la mesure de concentration d'endotoxines ;
- l'action mise en œuvre comprend l'arrêt du procédé ou une alerte en fonction d'une stabilité de ladite évolution temporelle de la mesure de concentration d'endotoxines ou d'une décroissance de la mesure de concentration d'endotoxines ;
- une relation d'étalonnage est déterminée pour chaque instant de mesure de la durée de mesure ;
- la relation d'étalonnage pour un instant de mesure est une relation d'étalonnage déterminée à partir des valeurs d'intensité lumineuse des chambres de référence d'un instant de mesure précédent ;
- l'évolution temporelle d'une mesure de concentration d'endotoxines dans l'échantillon biologique est déterminée plusieurs fois au cours de la durée de mesure, à chaque fois à la suite d'un instant de mesure pris en compte dans ladite évolution temporelle
- des réactifs d'analyse présents dans des chambres d'analyse et des réactifs de référence présents dans des chambres de référence comprennent un facteur C recombinant et un substrat fluorigène des chambres de référence comprenant des endotoxines à des concentrations prédéterminées ;
- au moins une chambre de référence est dépourvue d'endotoxine ;
- le support d'analyse comprend une pluralité de chambres d'analyse, et la détermination la détermination d'une valeur d'intensité lumineuse de chambre d'analyse pour ledit instant de mesure comprend la détermination d'une valeur statistiquement représentative de valeurs d'intensité lumineuse de plusieurs desdites chambres d'analyse.

L'invention concerne également un instrument d'analyse comprenant un imageur définissant un champ de vue, l'instrument étant configuré pour recevoir un support d'analyse dans le champ de vue de l'imageur, ledit support d'analyse comprenant au moins une chambre d'analyse configurée pour recevoir un échantillon biologique, et une pluralité de chambres de référence configurées pour recevoir un liquide de référence, les chambres de référence étant pourvues de réactifs de référence et de concentrations de référence, les réactifs de référence étant aptes à causer une réaction de luminescence en présence du liquide de référence en fonction de la concentration d'endotoxines dans la chambre de référence correspondante, la chambre d'analyse étant pourvue de réactifs d'analyse aptes à causer une réaction de luminescence en présence d'endotoxines de l'échantillon biologique, le système étant configuré pour mettre en œuvre au moins les étapes du procédé selon l'invention.

L'invention concerne également un système comprenant un instrument d'analyse et un support d'analyse selon l'invention.

**Présentation des figures**

D'autres caractéristiques, buts et avantages de l'invention ressortiront de la description qui suit, qui est purement illustrative et non limitative, et qui doit être lue en regard des dessins annexés sur lesquels :

la Figure 1 illustre schématique un support d'analyse disposé dans le champ de vue d'un imageur de l'instrument de mesure, selon un mode de réalisation possible de l'invention ;
la Figure 2 montre un exemple de support d'analyse comportant une pluralité de chambres, pouvant être utilisée pour la mise en place d'un échantillon biologique à analyser, selon un mode de réalisation possible de l'invention ;
la Figure 3 est un diagramme montrant des étapes du procédé selon un mode de réalisation possible de l'invention ;
la Figure 4 montre schématiquement des traitements appliqués à l'image d'une chambre selon un mode de réalisation possible ;
la Figure 5 montre un exemple d'évolution temporelle de valeurs d'intensité lumineuses de chambres de référence selon un mode de réalisation possible de l'invention ;
la Figure 6 montre des exemples de droites représentant des relations d'étalonnage liant valeur d'intensité lumineuse et concentration à cet instant de mesure, selon un mode de réalisation possible de l'invention ;

la Figure 7 montre une courbe illustrant un exemple d'évolution temporelle d'une concentration en analyte dans l'échantillon biologique au cours de la durée de mesure, selon un mode de réalisation possible de l'invention.

la Figure 8 montre une courbe illustrant un exemple d'évolution temporelle, au cours de la durée de mesure, du logarithme d'une moyenne de valeurs d'intensité lumineuse de chambres d'analyse accueillant l'échantillon biologique, selon un mode de réalisation possible de l'invention.

**Description détaillée**

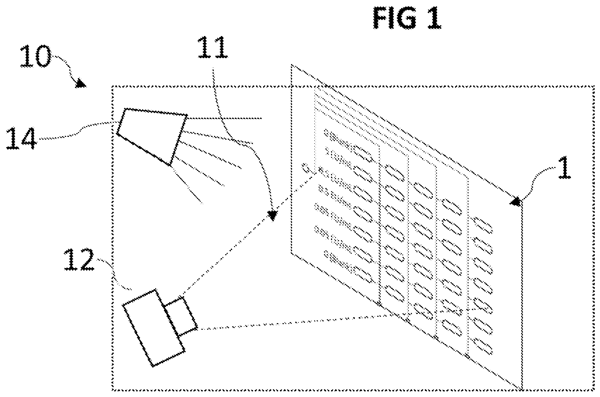

En référence à la Figure 1, le procédé d'analyse d'un échantillon biologique est mené au moyen d'un instrument d'analyse 10 comprenant un imageur 12, typiquement un fluorimètre, définissant un champ de vue 11, et d'un support d'analyse 1 introduit dans l'instrument d'analyse 10, dans le champ de vue 11 de l'imageur 12. Le support d'analyse 1 comprend au moins une chambre d'analyse configurée pour recevoir l'échantillon biologique, généralement sous forme liquide, et une pluralité de chambres de référence configurées pour recevoir un liquide de référence. L'instrument de mesure 10 peut également comprendre une source lumineuse 14 configurée pour éclairer le champ de vue 11 avec une lumière dont la longueur d'onde est susceptible de mettre en évidence un phénomène de fluorescence, c'est-à-dire de provoquer l'émission d'une lumière de fluorescence après excitation d'un fluophore. La longueur d'onde de la lumière d'illumination est donc choisie en fonction du fluophore à détecter, et plus particulièrement en fonction d'une longueur d'onde d'excitation du fluophore. De même, l'imageur 12 peut être pourvu d'un filtre de détection avec une bande passante optique correspondant à une longueur d'onde d'émission du fluophore à détecter. Sauf indication contraire, la source lumineuse éclaire le champ de vue 11 lors de l'acquisition des images.

L'instrument de mesure peut également comprendre des composants utilisés pour le traitement de données, tels qu'un processeur, une mémoire ou une alimentation électrique.

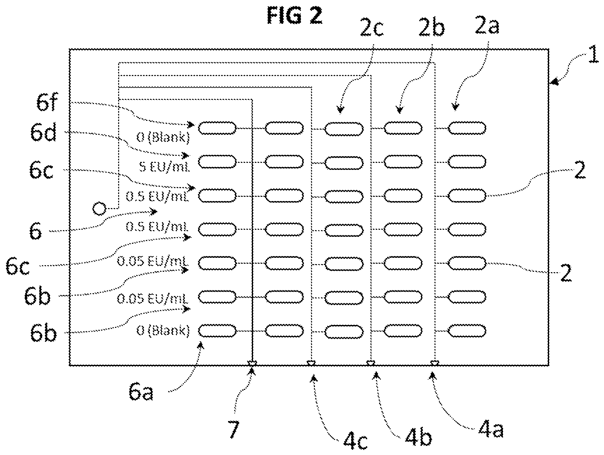

La Figure 2 montre un exemple de support d'analyse 1 comportant une pluralité de chambres d'analyse 2 pouvant être utilisée pour la mise en place d'un échantillon biologique à analyser. Les chambres d'analyse 2 sont ici organisées selon trois réseaux correspondant à trois dilutions différentes de l'échantillon. Dans l'exemple illustré, un premier réseau est défini par un premier canal d'alimentation 4a destiné à alimenter des premières chambres d'analyse 2a avec une première dilution, par exemple de 1:1, de l'échantillon biologique. Le premier réseau regroupe ainsi l'ensemble des premières chambres d'analyse 2a, lesquelles sont distribuées selon une direction, ici la direction verticale sur la figure 2. Un deuxième réseau est défini par un deuxième canal d'alimentation 4b destiné à alimenter des deuxièmes chambres d'analyse 2b avec une deuxième dilution, par exemple de 1:10, de l'échantillon biologique. Le deuxième réseau regroupe ainsi l'ensemble des deuxièmes chambres d'analyse 2b, lesquelles sont distribuées selon une direction, ici la direction verticale sur la figure 2. Un troisième réseau est défini par un troisième canal d'alimentation 4c destiné à alimenter des troisièmes chambres d'analyse 2b avec une troisième dilution, par exemple de 1:100, de l'échantillon biologique. Le troisième réseau regroupe ainsi l'ensemble des troisièmes chambres d'analyse 2c, lesquelles sont distribuées selon une direction, ici la direction verticale sur la figure 2. Si dans cet exemple trois dilutions sont utilisées, plus ou moins de dilutions peuvent être utilisées, définissant autant de réseaux de chambres d'analyse 2. De préférence toutefois, le support d'analyse comprend plusieurs chambres d'analyse 2, de préférence regroupées selon deux réseaux afin recevoir l'échantillon biologique selon deux dilutions différentes.

Le support d'analyse 1 comprend également une pluralité de chambres de référence 6 configurés pour recevoir un liquide de référence. Chaque chambre de référence 6 comprend des réactifs de référence préalablement disposés dans la chambre, ainsi que différentes concentrations d'endotoxines. De préférence toutefois, au moins une chambre de référence 6 est une chambre témoin ne comprenant pas d'endotoxine. De préférence, une matrice est alors présente dans la chambre témoin, avec un dépôt issu d'une solution permettant d'obtenir des caractéristiques chimiques proches de celles des endotoxines. Il s'agit par exemple d'un polyéther tel que le polyéthylène glycol (ou PEG), ou d'un polymère organique tel que le polyvinylpyrrolidone, ou PVP.

Les réactifs de référence sont aptes à causer une réaction de luminescence en fonction de la concentration d'endotoxines dans la chambre de référence correspondante. On entend par luminescence une émission de lumière sans incandescence, comme par exemple la fluorescence. Typiquement, ces réactifs de référence sont déshydratés, et les chambres de référence 6 sont configurées pour recevoir un liquide de référence permettant des réactions de se produire au contact des réactifs de référence. Typiquement, ce liquide de référence est une eau dépourvue d'endotoxine, plus connu sous le nom anglais de "*endotoxin-free water*". Une telle eau dépourvue d'endotoxine répond généralement à des spécificités autres que l'absence d'endotoxine, telle qu'une stérilité garantie, un filtrage à moins de 1 $\mu$m, etc.. Les différentes concentrations d'endotoxines dans les chambres de référence 6 en contenant couvrent typiquement une plage de concentrations avec des facteurs de 1 à plusieurs dizaines, voire de 1 à 100. De préférence, l'endotoxine est une LipoPolySaccharide (LPS) produite uniquement par les bactéries à Gram négatif, telles que Escherichia coli, Salmonella enteritidis, Legionella pneumophila, Campylobacter jejuni, Vibrio cholerae, Shigella dysenteriae, Pseudomonas aeruginosa et bien d'autres encore.

Des chambres de référence 6 distinctes sont pourvues d'au moins deux concentrations d'endotoxines différentes, et de préférence d'au moins trois concentrations d'endotoxines différentes. Les réactifs de référence peuvent comprendre un tampon, un facteur C recombinant et un substrat fluorigène. Les réactifs dans les chambres de référence 6 comprennent par exemple un agent de détection dans un état non actif en l'absence d'activation, qui peut comprendre les endotoxines, un agent d'activation de l'agent de détection comprenant une enzyme et un substrat fluorigène, et un réactif de contrôle adapté pour contrôler la fonctionnalité du réactif de détection.

Dans l'exemple illustré, un canal d'alimentation 7 est configuré pour alimenter les chambres de référence 6 en fluide de référence. Le canal d'alimentation 7 s'étend selon une direction, ici la direction verticale, le long de laquelle sont distribuées les chambres d'alimentation. Dans cet exemple, les chambres de référence 6 sont disposées par paires, une chambre de référence 6 de chaque côté du canal 7, formant ainsi deux colonnes de chambres de référence 6. Les chambres de référence 6 d'une paire sont pourvues d'une même concentration d'endotoxines. Dans l'exemple, en partant du bas vers le bas de la figure 2, la première paire est constituée de deux chambres témoins 6a désignées comme "blank", dépourvues d'endotoxine, la deuxième paire et la troisième paire sont constituées chacune de deux chambres de référence 6b, chaque chambres de référence 6b de la deuxième paire et la troisième paire étant pourvues d'une première concentration d'endotoxines de 0,05 EU/mL, EU désignant l'unité endotoxine correspondant à une unité internationale correspondant à 100 pg d'endotoxines, la quatrième paire et la cinquième paire sont constituées chacune de deux chambres de référence 6c, chaque chambres de référence 6c de la quatrième paire et la cinquième paire étant pourvues d'une deuxième concentration d'endotoxines de 0,5 EU/mL, la sixième paire est constituée de deux chambres de référence 6d pourvues d'une troisième concentration d'endotoxines de 5 EU/mL, la septième paire est constituée de deux chambres témoins 6f désignées comme "blank", et dépourvues d'endotoxine.

Les chambres d'analyse 2 et les chambres de référence 6 présentent une même configuration. Typiquement, ces chambres 2, 6 présentent au moins une paroi transparente pour les longueurs d'onde susceptibles d'être émises lors des réactions, cette paroi étant visible par l'imageur. Les chambres d'analyse 6 sont pourvues de réactifs d'analyse aptes à causer une réaction de luminescence en présence d'endotoxines dans l'échantillon biologique avec lequel lesdits réactifs sont mis en contact. Les réactifs d'analyse peuvent être identiques aux réactifs de référence. Les réactifs comprennent, par exemple un agent de détection dans un état non actif en l'absence d'activation dépourvu d'endotoxines pour les chambres d'analyse 2 et éventuellement certaines chambres de référence 6 (par exemple les chambres témoins), un agent d'activation de l'agent de détection comprenant une enzyme et un substrat fluorigène, et un réactif de contrôle adapté pour contrôler la fonctionnalité du réactif de détection.

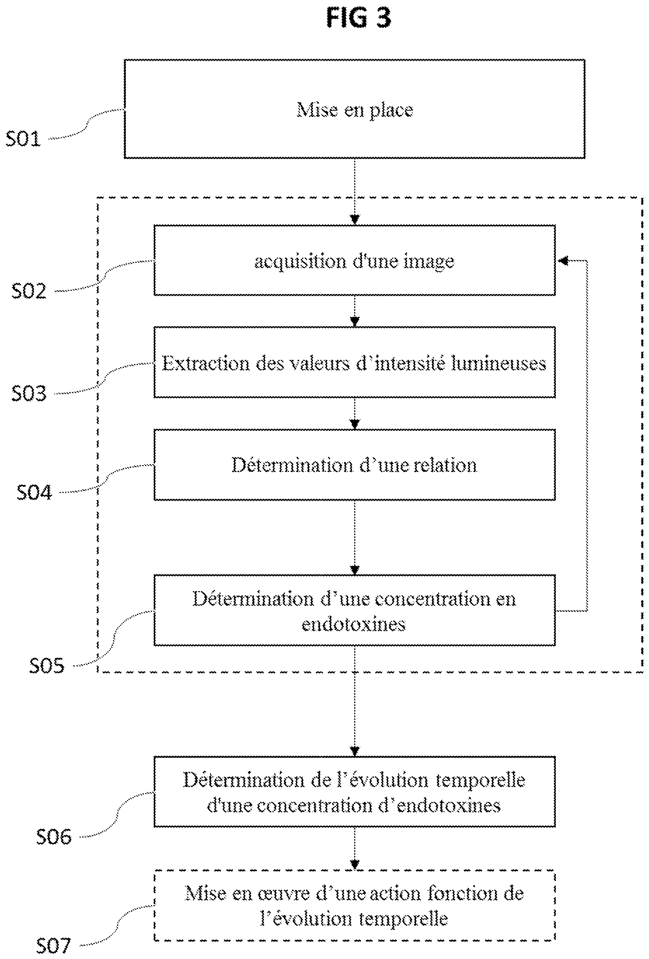

En référence à la Figure 3, lors d'une première étape de mise en place S01, l'échantillon biologique est mis en place dans les chambres d'analyse 2, typiquement en alimentant les chambres d'analyse 2 par les canaux d'alimentation 4a, 4b, 4c. Le liquide de référence est également mis en place dans les chambres de référence 6 au moyen du canal d'alimentation 7. Le support d'analyse 1 est ensuite introduit dans l'instrument d'analyse 10, dans un champ de vue 11 de l'imageur 12 du fluorimètre. Typiquement, l'instrument d'analyse 10 assure le maintien de certaines conditions prédéterminées telles que le maintien de l'échantillon biologique à une température donnée (par exemple 37°C).

Ensuite, plusieurs instants de mesure d'une durée de mesure sont mis en œuvre lors des étapes qui vont être décrites plus bas. Il est évident que l'instrument de mesure comporte des moyens de traitement de données tel qu'un processeur, une mémoire, et une interface d'entrée sortie qui ne seront pas décrites en détail. La durée de mesure s'entend typiquement du temps s'écoulant entre la mise en place du support d'analyse 10 contenant l'échantillon biologique dans le champ de vue 11 et la dernière mesure par acquisition d'image, avant l'interruption de la mesure et la fourniture des résultats. La durée de mesure s'étend sur plusieurs minutes, et généralement sur plusieurs dizaines de minutes, par exemple plus de 20 ou 40 minutes. Les instants de mesure sont répartis dans la durée de mesure, typiquement avec une périodicité de quelques minutes, par exemple toutes les minutes ou toutes les deux minutes. Une durée de mesure comprend de préférence au moins 5 instants de mesure, et de préférence comprend au moins 10 instants de mesure. Il est à noter que la durée de mesure peut comprendre des acquisitions d'images et des mesures qui ne font pas partie des instants de mesure au sens de l'invention, dès lors que des étapes qui vont être décrites ne sont pas mises en œuvre. En particulier, une acquisition d'image initiale peut être effectuée au début de la durée de mesure visant à déterminer une image de référence destinée au traitement des autres images.

Lors de chaque instant de mesure, une image est acquise (étape S02) par l'imageur 12. L'image est ici une image bidimensionnelle formée de pixels spatialement organisés par des coordonnées auxquels sont associés des valeurs d'intensité lumineuse. Comme le support d'analyse 1 est dans le champ de vue 11 de l'imageur 12, il s'agit d'une image du support d'analyse. De préférence, le support d'analyse 1 remplit l'ensemble de l'image acquise. Par ailleurs, l'image acquise peut représenter tout le support d'analyse 1, ou du moins l'ensemble des chambres 2, 6 du support d'analyse 1, ou bien peut représenter seulement certaines des chambres 2, 6, auquel cas il est possible d'acquérir plusieurs images en déplaçant le champ de vue 11 entre deux acquisitions relativement au support d'analyse 1 afin d'imager toutes les chambres 2, 6 qui doivent l'être, dans un intervalle de temps s'interprétant comme un instant de mesure. Dans la mesure où acquérir une ou plusieurs images ne change pas fondamentalement le procédé, il sera par la suite fait référence uniquement à l'acquisition d'une image par soucis de simplicité non restrictive.

Des valeurs d'intensité lumineuse de chambres de référence 6 et/ou de chambres d'analyse 2 sont ensuite déterminées à partir de l'image acquise (étape S03). Afin d'améliorer les résultats de cette détermination des valeurs d'intensité lumineuse, il est possible de mettre en œuvre une ou plusieurs étapes de pré-traitement de l'image acquise.

Une première étape de pré-traitement peut être l'application d'une ou plusieurs corrections des valeurs d'intensité des pixels de l'image acquise, au moyen soit de données de correction prédéterminées avant le procédé et communes à toutes les mise en œuvre du procédé, soit de données de correction déterminées au début de la durée de mesure et donc spécifiques à cette mise en œuvre du procédé. Typiquement, les données de correction prennent la forme d'une matrice de valeurs de la taille de l'image, et la correction s'effectue en soustrayant ou multipliant une valeur d'intensité lumineuse d'un pixel par une valeur de la matrice de correction.

En particulier, des données de correction peuvent correspondre à une image sombre, c'est-à-dire à une image du champ de vue 11 acquise en l'absence d'éclairage de celui-ci. L'obscurité du champ de vue 11 a pour conséquence que n'apparaissent alors que les bruits causés par les courants d'obscurité ou autres perturbations similaires. Les valeurs d'intensité lumineuse de l'image sombre sont soustraites des valeurs d'intensité de l'image acquise afin de supprimer ces bruits. Les données de correction peuvent être des données d'homogénéisation, visant en particulier à corriger une éventuelle inhomogénéité de l'éclairage du support d'analyse 1 par l'instrument d'analyse 10 ou d'éventuelles autres homogénéités, par exemple optiques. Ces données d'homogénéisation peuvent notamment prendre la forme d'une matrice de correction dérivée d'une image de fond acquise lorsqu'un objet avec une réflexion ou une fluorescence spatialement uniforme, comme par exemple une feuille d'aluminium, est présent dans le champ de vue 11 et est éclairé. La matrice de correction peut alors contenir des valeurs permettant de corriger d'éventuelles inhomogénéités ainsi détectées.

Une autre correction peut être de soustraire aux valeurs d'intensité de l'image acquise les valeurs d'intensité lumineuse correspondantes d'une image initiale, acquise au début de la durée de mesure, avec le support d'analyse 1 disposé dans le champ de vue 11. Dans la mesure où cette image initiale correspond à un instant pour lequel les réactions de fluorescence n'ont pas encore débuté, une telle correction permet de mettre en évidence dans l'image acquise du support d'analyse 1 les seules variations lumineuses dues à ces réactions de fluorescence et donc s'affranchir des défauts tels que les poussières présentes sur l'objet générant un signal de fluorescence parasite. En d'autres termes, cela permet de ramener les valeurs d'intensité à une valeur relative par rapport à une valeur initiale, celle-ci étant à zéro dans l'image initiale du début de la durée de mesure.

Une fois ces éventuels prétraitements effectués, il peut être procédé à l'extraction des valeurs d'intensité lumineuse des chambres 2, 6 dans l'image acquise (étape S03). De préférence, à la fois les valeurs d'intensité lumineuses des chambres de référence 6 et les valeurs d'intensité lumineuses de la ou des chambres d'analyse 2 sont déterminées à chaque instant de mesure. Il est toutefois possible à un instant de mesure de n'extraire que les valeurs d'intensité lumineuses des chambres d'analyse 2, par exemple lorsqu'il n'est pas nécessaire de déterminer la relation d'étalonnage qui sera discutée plus bas à cet instant de mesure.

Dans la mesure où l'image acquise représente plusieurs chambres 2, 6 (par exemple 35 dans l'exemple de la Figure 2), il convient de localiser chaque chambre 2, 6 dans l'image acquise. Il est possible de considérer qu'à chaque chambre 2, 6 correspond un emplacement prédéterminé, dicté par l'organisation spatiale du support d'analyse 1 et par sa disposition connue dans le champ de vue 11. Toutefois, une telle approche nécessite un positionnement précis du support d'analyse 1 et un réglage fin, et est susceptible de présenter des erreurs si le positionnement réel du support d'analyse 1 n'est pas celui attendu. Il est par conséquent préférable de mettre en œuvre une localisation des chambres 2, 6 dans l'image acquise, par exemple au moyen d'une reconnaissance de forme utilisant un modèle de forme (ou "template") correspondant à celle des chambres. Une fonction d'inter-corrélation impliquant le modèle de forme est appliquée à l'image acquise, déterminant un score de similarité permettant de déterminer la position du modèle dans l'image, et donc des chambres 2, 6 recherchées.

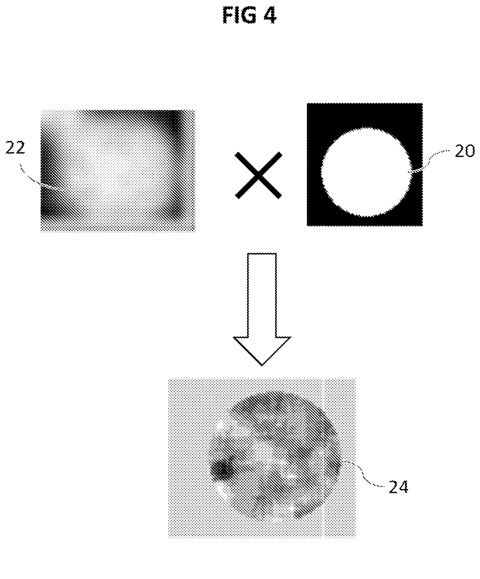

Il peut être procédé à un masquage de l'image au moyen d'un masque visant à ne conserver de l'image que les zones des chambres qui doivent être traitées. Typiquement, le masque présente des pixels avec une valeur de 1 pour les zones de l'image à conserver, et 0 pour le reste de l'image. Sur l'exemple de la Figure 4, le masque 20 comprend une zone en forme de disque à conserver, en blanc (valeur 1), tandis que le reste du masque 20 est noir (valeur 0). Le masque 20 illustré ici ne recouvre qu'une zone réduite destinée à être centrée sur un emplacement 22 identifié d'une chambre 2, 6, celles-ci étant traitées une par une, mais le masque 20 pourrait recouvrir plusieurs chambres 2, 6. L'application du masque 20 peut consister à multiplier les valeurs d'intensités des pixels de chaque emplacement 22 d'une chambre avec les valeurs correspondantes du masque 20. On obtient alors uniquement la zone 24 d'une chambre 2, 6 qui doit être traitée, en forme de disque dans cet exemple, les pixels en-dehors de ces zones 24 étant nuls et n'étant pas pris en compte ensuite.

Une valeur d'intensité d'une chambre 2, 6 est ensuite dérivée des valeurs d'intensité des pixels de la chambre ainsi isolés, de sorte à être statistiquement représentative de celles-ci, par exemple en calculant une moyenne, une médiane, ou un percentile.

Puisque le support comprend généralement plusieurs chambres d'analyse 2, en particulier plusieurs chambres d'analyse 2 pourvues des mêmes réactifs d'analyse, une valeur statistiquement représentative de valeurs d'intensité lumineuse de plusieurs desdites chambres d'analyse 2 peut être déterminée, à partir de laquelle est dérivée une valeur d'intensité lumineuse de chambre d'analyse pour la suite du procédé. Cette valeur statistiquement représentative peut typiquement être une mesure de tendance centrale telle que la moyenne ou de préférence la médiane des valeurs d'intensité lumineuse desdites plusieurs chambres d'analyse 2.

Il est possible de mettre en œuvre une exclusion des valeurs aberrantes, susceptibles de correspondre à des dysfonctionnements, comme par exemple un problème de remplissage avec l'échantillon biologique ou le liquide de référence, la présence d'une bulle d'air ou d'une poussière, etc. C'est notamment parce que des dysfonctionnements peuvent se produire que le support d'analyse 1 comporte de préférence plusieurs chambres d'analyse 2, de préférence au moins trois chambres d'analyse 2, et qu'à chaque concentration d'endotoxines de référence sont associées plusieurs chambres de référence 6, de préférence au moins trois chambres de référence avec une même concentration d'endotoxines. L'exclusion des valeurs aberrantes peut par exemple comprendre la comparaison par rapport à un seuil d'un critère de déviance de chaque chambre 2, 6, celui-ci étant de préférence dépendant des valeurs des valeurs d'intensité des autres chambres, au moins celles de même configuration (chambre d'analyse 2 ou chambres de référence 6 de même concentration d'endotoxines), par exemple prenant en compte une mesure de tendance centrale comme la moyenne ou de préférence la médiane. Le seuil peut par exemple être un écart par rapport à la mesure de tendance centrale. Les valeurs d'intensité lumineuse dépassant le seuil d'un critère de déviance peuvent être écartées et ne pas être prise en compte pour la suite.

Une fois les valeurs d'intensité lumineuse des chambres de référence 6 extraites, il est établi une relation d'étalonnage liant valeur d'intensité lumineuse et concentration d'endotoxines à partir des valeurs d'intensité lumineuse des chambres de référence 6 extraites de l'image acquise et des images acquises précédentes. Si plusieurs chambres de référence 6 correspondent à une même concentration d'endotoxines, comme par exemple les chambres témoins 6a ou les quatre chambres de référence 6 avec une concentration de 0,05 EU/mL dans l'exemple, les valeurs d'intensité de ces chambres de référence 6 peuvent être combinées, par exemple via une valeur statistiquement représentative de ces valeurs d'intensité, comme par exemple une mesure de tendance centrale comme la moyenne ou de préférence la médiane.

La relation d'étalonnage prend la forme d'une fonction qui fait correspondre une concentration d'endotoxine à une valeur d'intensité lumineuse. Dans l'exemple qui suit, la relation d'étalonnage relie un logarithme des valeurs d'intensité lumineuse au logarithme de la concentration d'endotoxines. Le fait de prendre en compte des logarithmes permet à la relation d'étalonnage d'être plus robuste aux erreurs. Il est toutefois possible d'utiliser d'autres types de relation d'étalonnage, comme par exemple une relation affine liant directement entre la valeur d'intensité lumineuse et concentration d'endotoxines. D'autres types de régression peuvent être utilisées pour déterminer la relation d'étalonnage, comme par exemple une régression non linéaire ou bien encore une régression paramétrique ou non paramétrique. Le choix du type de relation d'étalonnage est effectué pour refléter au mieux la relation physique entre la valeur d'intensité lumineuse et la concentration d'endotoxines, et peut donc dépendre des réactifs utilisés et de leurs cinétiques.

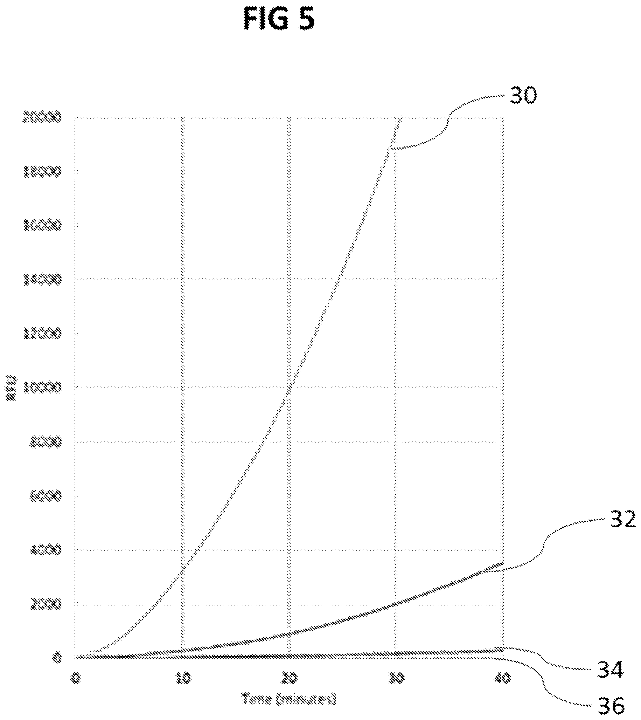

Toutefois, la relation entre valeur d'intensité lumineuse et concentration d'endotoxines varie au cours du temps, en raison des cinétiques des réactions impliquées dans l'obtention de la fluorescence. La Figure 5 montre des exemples d'évolution temporelle des valeurs d'intensité en RFU, de l'anglais "Relative Fluorescence Unit" pour Unité de Fluorescence Relative pour des chambres de référence 6 associées aux concentrations d'endotoxines 5 EU/mL (courbe 30), 0,5 EU/mL (courbe 32), et 0,05 EU/mL (courbe 34), et 0 EU/mL (i.e. les chambres témoins 6a, courbe 36). La RFU est fonction de l'intensité (quantité de photons collectés par l'imageur 12) par rapport à une référence, la RFU augmentant avec l'intensité. Il est possible que les valeurs d'intensité lumineuse des chambres témoins 6a soient non nulles, et reflètent donc des fluctuations dues à des perturbations (faibles et donc non visibles sur la figure). Il est donc possible de soustraire les valeurs d'intensité lumineuse des chambres témoins 6a de celles associées aux différentes concentrations d'endotoxines. Les autres courbes 30, 32, 34 montrent clairement que les évolutions temporelles des valeurs d'intensité lumineuse, si elles sont toutes croissantes, présentent des cinétiques très différentes en fonction de leurs concentrations en endotoxines respectives. Une relation d'étalonnage liant valeur d'intensité lumineuse et concentration d'endotoxines n'est donc valable qu'à l'instant de mesure des données à partir desquelles cette relation est établie.

C'est donc afin de prendre en compte ces différences cinétiques qu'une relation d'étalonnage liant valeur d'intensité lumineuse et concentration est déterminée (S04) pour chacun instant d'une pluralité d'instants de mesure, à partir des valeurs d'intensité lumineuse des chambres de référence 6 à ces instants de mesure, pour pouvoir être exploitable pour les données de cet instant de mesure ou les instants de mesure qui suivent. Lors d'un instant de mesure, sont ainsi connues les valeurs d'intensité lumineuse pour chaque concentration d'endotoxine de référence (par exemple 5 EU/mL, 0,5 EU/mL, 0,05 EU/mL). Il est donc possible d'établir une telle relation, typiquement par approximation d'une fonction. Par exemple, une régression linéaire peut être utilisée, ou bien encore une interpolation. De préférence, la relation d'étalonnage lie plus précisément un logarithme de valeur d'intensité lumineuse et un logarithme de concentration d'endotoxines. Par exemple, la relation d'étalonnage peut lier le logarithme de valeur d'intensité lumineuse et le logarithme de concentration d'endotoxines en une fonction linéaire affine.

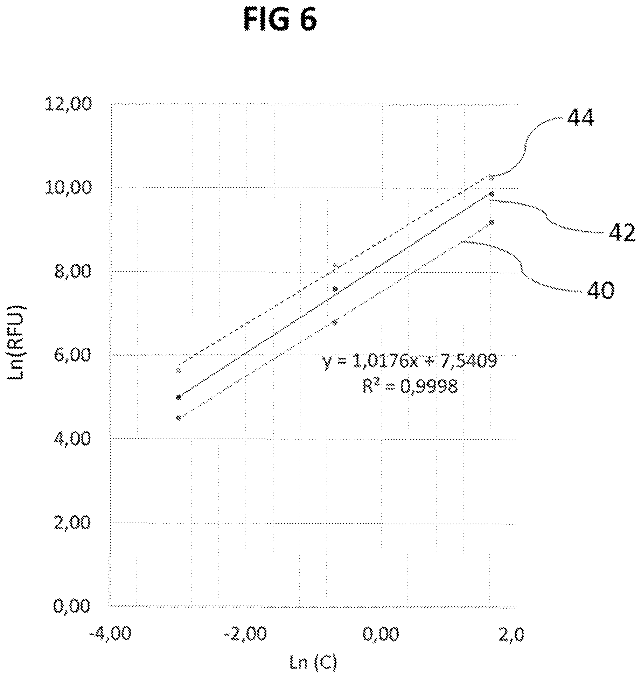

La Figure 6 montre trois droites 40, 42, 44 illustrant des fonctions affines reliant le logarithme des valeurs d'intensité lumineuse (axe y) au logarithme de la concentration C en endotoxines (axe x). Ces droites 40, 42, 44 ont été obtenues par régression linéaire pour trois instants de mesure (20 minutes, 30 minutes, 40 minutes) avec les valeurs connues de concentration d'endotoxines des chambres de référence 6 (C=5 EU/mL, C=0,5 EU/mL, et C=0,05 EU/mL). La première droite 40 (en pointillés) correspond à la relation à 20 minutes, la deuxième droite 42 (en trait plein) correspond à la relation à 30 minutes, et la troisième droite 44 (en tirets) correspond à la relation à 40 minutes. Dans cet exemple, l'approximation par régression linéaire fait que la relation d'étalonnage est de la forme $\ln(\text{Val}_{RFU}) = a(t) \times \ln(c) + b(t)$, avec $\text{Val}_{RFU}$ la valeur d'intensité lumineuse, $a(t)$ le coefficient directeur à l'instant $t$ et $b(t)$ une constante réelle à l'instant $t$. Par exemple, la première droite 40 (à t=20 minutes) a pour équation $y = 1,0176\, x + 7,5409$, pour un coefficient de détermination de 0,9998. Il apparaît ici clairement que la relation d'étalonnage n'est pas la même selon l'instant de mesure. On constate non seulement un décalage global vers des valeurs d'intensité lumineuse plus élevées au cours du temps (décalage de la constante $b(t)$ au cours du temps), mais également une variation du coefficient directeur $a(t)$ des droites.

Idéalement, une relation d'étalonnage liant la valeur d'intensité lumineuse et concentration d'endotoxines spécifique est déterminée à chaque instant de mesure, c'est-à-dire à chaque fois qu'une image du support d'analyse est acquise (étape S02). Il est toutefois possible de ne déterminer la relation d'étalonnage que pour certains instants de mesure de la durée de mesure, et pas pour d'autres instants de mesure de la durée de mesure. Par exemple, la relation d'étalonnage peut n'être déterminée que tous les deux ou trois instants de mesure, ou avec une fréquence variant avec l'avancement de la durée de mesure, et en particulier avec une fréquence plus élevée au début de la durée de mesure qu'à la fin de la durée de mesure. La détermination d'une relation d'étalonnage peut par exemple être effectuée au moins pour des instants de mesure au début de la durée de mesure, puis ne plus être effectuée si un critère de stabilité est rempli.

Par exemple, un tel critère de stabilité peut être une linéarité suffisante de la relation d'étalonnage (par exemple entre des logarithmes), typiquement en comparant un terme d'erreur de linéarisation avec un seuil. D'autres critères peuvent être utilisés, notamment en fonction du type de relation d'étalonnage, comme par exemple des limites pour des coefficients de corrélation ou d'autres paramètres.

Une relation d'étalonnage spécifique est déterminée pour au moins 3 instants de mesure, de préférence pour au moins 5 instants de mesure, et de préférence encore pour au moins 8 instants de mesure parmi les instants de la durée de mesure. Une relation d'étalonnage spécifique est déterminée au moins pour des instants de mesure répartis sur une durée d'au moins 3 minutes, de préférence d'au moins 5 minutes, et de préférence encore d'au moins 8 minutes. De préférence, une relation d'étalonnage spécifique est déterminée pour au moins le quart des instants de mesure, et de préférence pour au moins la moitié des instants de mesure.

Lorsqu'une relation d'étalonnage spécifique est déterminée à un instant de mesure, et en particulier lorsque ladite relation d'étalonnage a rempli un critère de stabilité prédéfini, il est possible d'utiliser cette même relation d'étalonnage pour d'autres instants de mesure subséquents. Dans tous les cas, une relation d'étalonnage est disponible pour chaque instant de mesure, qu'elle soit spécifiquement déterminée pour cet instant de mesure où qu'elle soit héritée d'une détermination en lien avec un instant de mesure précédent. Et tous les instants de mesure de la durée de mesure n'ont pas la même relation d'étalonnage.

Pour chaque instant de mesure, la relation d'étalonnage permet déterminer au moins une mesure de concentration d'endotoxines dans l'échantillon biologique à partir de la valeur d'intensité associée aux chambres d'analyse audit instant de mesure (étape S05). En effet, la relation d'étalonnage relie valeur d'intensité lumineuse et concentration d'endotoxines, de sorte qu'en mesurant une valeur d'intensité lumineuse, on retrouve une mesure de concentration d'endotoxines correspondant pour cet instant. Par exemple, en reprenant une relation d'étalonnage de la forme $\ln(\text{Val}_{RFU}) = a(t) \times \ln(c) + b(t)$, celle-ci peut s'écrire:

$$\ln(C) = \frac{\ln(\text{Val}_{RFU}) - b(t)}{a(t)}$$

et la concentration d'endotoxines c de l'échantillon recherchée à l'instant t est donc

$$C(t) = e^{\frac{\ln(\text{ValRFU}) - b(t)}{a(t)}}$$

Bien entendu, l'expression de la concentration d'endotoxines en fonction de la valeur d'intensité lumineuse dépend directement de l'expression de la relation d'étalonnage, et peut donc prendre une tout autre forme.

Cette détermination de la concentration d'endotoxines dans l'échantillon étant réalisée pour plusieurs instants de mesure au cours de la durée de la mesure, la concentration d'endotoxines est précisément connue à chacun de ces instants de mesure.

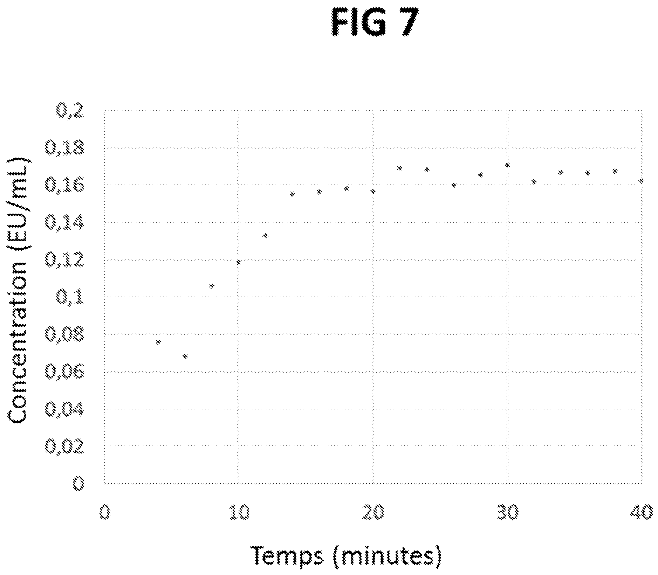

Il est alors possible de déterminer une évolution temporelle d'une mesure de concentration d'endotoxines dans l'échantillon biologique au cours de la durée de mesure (étape S06). La Figure 7 montre un exemple d'évolution temporelle de la mesure de concentration d'endotoxines dans l'échantillon (en Eu/mL), en fonction du temps (en minutes), obtenu en utilisant, pour chaque instant de mesure (représentés par des points), une relation d'étalonnage spécifique de cet instant de mesure. Il est notamment possible de constater une stabilisation de la concentration d'endotoxines au bout de 15 minutes. Plus précisément, les premières minutes correspondent à un régime transitoire dans lequel se produit des effets de re-suspension et d'homogénéisation des réactifs dans l'échantillon liquide présent dans les chambres d'analyse 2, tandis qu'ensuite s'organisent les réactions enzymes-substrat, aboutissant un régime stabilisé de ces réactions, se traduisant par la stabilisation constatée de la valeur mesurée de concentration d'endotoxines.

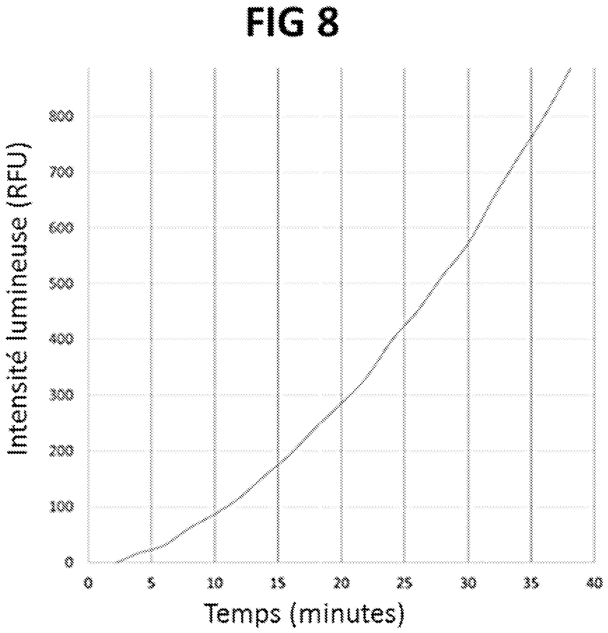

A titre de comparaison, la Figure 8 montre l'évolution temporelle des valeurs d'intensité lumineuse pour l'exemple de la Figure 7. Plus précisément, la courbe de la Figure 8 illustre l'évolution temporelle, au cours de la durée de mesure, du logarithme de la moyenne de valeurs d'intensité lumineuse de sept chambres d'analyse 2 accueillant l'échantillon biologique. Contrairement à l'évolution temporelle de la mesure de concentration d'endotoxines de la Figure 7, les valeurs d'intensité lumineuse présentent une croissance continue au cours du temps. Or, l'intensité de la fluorescence n'est pas directement représentative de la concentration d'endotoxines. C'est plutôt la cinétique respective d'augmentation d'intensité lumineuse qui est liée à la concentration d'endotoxines. La stabilisation de la valeur mesurée de concentration d'endotoxines est reflétée par la stabilisation de la pente sur la courbe de la Figure 8, par rapport à la relation d'étalonnage.

Les seules valeurs des valeurs d'intensité lumineuse ne reflètent donc pas la stabilisation constatée au bout des 15 minutes. Seul le procédé proposé, avec une relation d'étalonnage évoluant avec les instants de mesure et donc le temps, permet de rendre compte de cette stabilisation.

La détermination de l'évolution temporelle de la mesure de concentration d'endotoxines peut être faite uniquement à la fin de la durée de mesure, à partir de la masse d'instants de mesure. Toutefois, cette détermination de l'évolution temporelle de la mesure de concentration d'endotoxines dans l'échantillon peut être effectuée dès lors que l'on dispose des valeurs d'intensité lumineuse des chambres de référence et d'analyse pour au moins deux instants de mesure. Par conséquent, la détermination de l'évolution temporelle de la mesure de concentration d'endotoxines est réalisée plusieurs fois au cours de la durée de mesure, à la suite de plusieurs instants de mesure. On réalise ainsi une sorte de mise à jour de l'évolution temporelle de la mesure de concentration d'endotoxines. La détermination de l'évolution temporelle peut notamment être effectuée après chaque instant de mesure dès lors que l'on dispose des valeurs d'intensité lumineuse des chambres de référence et d'analyse pour au moins deux instants de mesure, ou une fois qu'un nombre déterminé d'instants de mesure ont été atteints. Cette détermination peut également être périodique, avec une période supérieure à l'écart entre deux instants de mesure.

La connaissance de l'évolution temporelle de la mesure de concentration d'endotoxines dans l'échantillon présente plusieurs avantages. Par exemple, elle permet de détecter un éventuel dysfonctionnement si cette évolution temporelle présente des anomalies, comme par exemple une décroissance prononcée, qui ne seraient peut-être pas détectées avec une seule mesure isolée à la fin de la durée de mesure. Le fait de mettre à jour l'évolution temporelle de la mesure de concentration d'endotoxines permet d'ailleurs de détecter de telles anomalies avant la fin de la durée de mesure initialement prévue, et ainsi de stopper l'analyse de l'échantillon, par exemple pour la recommencer, permettant de gagner un temps important.

A l'instar de l'exemple de la Figure 7, il est également possible de détecter une stabilisation de la concentration d'endotoxines (par exemple avec des variations inférieures à un seuil donné) sur plusieurs instants de mesure (par exemple sur 3 à 6 instants de mesure, ou sur une durée d'au moins 3 minutes, de préférence d'au moins 5 minutes), et de procéder alors à l'arrêt de l'analyse de l'échantillon. Dans l'exemple de la figure 7, un arrêt de la mesure au bout de 20 minutes aurait permis de diviser par deux la durée de mesure, et donc d'accélérer d'autant la quantification d'endotoxines de l'échantillon biologique.

Il est ainsi possible de mettre en œuvre une action fonction de l'évolution temporelle de la mesure de concentration d'endotoxines (étape S07), comme par exemple en cas d'anomalie ou de stabilisation de la concentration, l'arrêt de la mesure et/ou le déclenchement d'une alerte à l'opérateur (indication visuelle ou sonore par exemple), ou toute autre action permettant d'exploiter la connaissance de l'évolution temporelle de la mesure de concentration d'endotoxines dans l'échantillon.

Comme évoqué précédemment, la stabilisation de l'évolution temporelle de la mesure de concentration d'endotoxines, à un niveau suffisamment élevé, peut montrer que les réactions attendues ont entièrement eut lieu, et qu'un prolongement de la durée de mesure n'est pas nécessaire. L'arrêt de la mesure est donc déclenché en fonction de l'évolution temporelle de la mesure de concentration d'endotoxines, et non pas en fonction d'un temps prédéterminé. Typiquement, les conditions pour arrêter la mesure sur ce critère de stabilité comprennent des valeurs de concentration d'endotoxines présentant entre elles des variations inférieures à un seuil de variation, par exemple sur les 3 à 6 derniers instants de mesure, ou sur les instants de mesure d'une durée prédéfinie, comme par exemple une durée d'au moins 3 minutes, de préférence d'au moins 5 minutes. Cette approche permet de minimiser la durée requise pour obtenir des résultats de mesure, en fonction d'un écart de quantification acceptable.

En l'absence de stabilisation de l'évolution temporelle de la mesure de concentration d'endotoxines, la mesure se poursuit avec de nouveaux instants de mesure jusqu'à ce que cette stabilité soit atteinte ou qu'une limite de temps soit atteinte. En l'absence de cette stabilité après un certain temps, la mesure peut être stoppée et/ou une alerte peut être émise. Un dysfonctionnement peut ainsi être mis en évidence plus rapidement.

La validité de la quantification d'endotoxines peut être affectée par l'absence de stabilité de l'évolution temporelle de la mesure de concentration d'endotoxines, et notamment les résultats peuvent être invalidés du fait de cette absence. Cela permet d'éviter de prendre en compte des résultats finaux de quantification d'endotoxines qui pourraient se révéler erronés en raison d'un dysfonctionnement mis en évidence par l'absence de stabilité.

Il en va de même pour une décroissance prononcée de la mesure de concentration d'endotoxines, qui met en évidence un dysfonctionnement. Même lors d'un régime stabilisé comme celui atteint sur la Figure 7, la mesure de concentration d'endotoxines montrent de légères fluctuations qui sont normales. En revanche, il serait anormal de constater une décroissance forte de cette mesure de concentration d'endotoxines. Il est donc possible d'utiliser un seuil maximal de décroissance pour détecter un tel comportement anormal, dont le franchissement peut causer l'arrêt du procédé ou une alerte en fonction d'une décroissance de la mesure de concentration d'endotoxines.

L'évolution temporelle de la mesure de concentration d'endotoxines peut être aussi utiliser pour montrer l'absence des réactions attendues, par exemple en l'absence d'endotoxine dans l'échantillon biologique, en vérifiant que la mesure de la concentration d'endotoxines ne s'est pas élevée de manière transitoire au cours de la durée de mesure.

Bien entendu, il est possible de prévoir un seuil minimal pour les valeurs de concentration d'endotoxines, permettant de s'assurer que des réactions ont bien eu lieu, pour la mise en œuvre de certaines des actions précitées.

A la fin de la durée de mesure, que celles-ci interviennent en raison de la stabilité de la mesure de concentration d'endotoxines, de l'expiration d'un temps imparti, ou de la détection d'une anomalie, il est alors possible de fournir des résultats d'analyse dérivés de l'évolution temporelle de la concentration d'endotoxines, avant l'arrêt du procédé ou au moment de l'arrêt du procédé. Il est à noter que l'action fonction de l'évolution temporelle peut également être la poursuite de la mesure si l'évolution temporelle ne présente pas de motif d'arrêt ou d'alerte.

L'invention n'est pas limitée au mode de réalisation décrit et représenté aux figures annexées. Des modifications restent possibles, notamment du point de vue de la constitution des diverses caractéristiques techniques ou par substitution d'équivalents techniques, sans sortir pour autant du domaine de protection de l'invention.

**Revendications**

1.  Procédé de quantification d'endotoxines d'un échantillon biologique par un instrument d'analyse (10) comprenant un imageur (12) définissant un champ de vue (11), un support d'analyse (1) étant introduit dans le champ de vue (11) de l'instrument d'analyse (10), ledit support d'analyse (1) comprenant au moins une chambre d'analyse (2) configurée pour recevoir ledit échantillon biologique et une pluralité de chambres de référence (6) configurées pour recevoir un liquide de référence, le procédé comprenant d'abord une mise en place (S01) de l'échantillon biologique dans ladite chambre d'analyse (2) et du liquide de référence dans la pluralité de chambres de référence (6), des chambres de référence (6) étant pourvues de réactifs de référence et de différentes concentrations d'endotoxines, lesdits réactifs de référence étant aptes à causer une réaction de luminescence en présence du liquide de référence en fonction de la concentration d'endotoxines dans la chambre de référence (6) correspondante, la chambre d'analyse (2) étant pourvue de réactifs d'analyse aptes à causer une réaction de luminescence en présence d'endotoxines de l'échantillon biologique,

    le procédé comprenant, pour chaque instant de mesure d'une durée de mesure, l'acquisition (S02) audit instant de mesure d'une image du support d'analyse (1) et la détermination (S03), à partir de ladite image du support d'analyse (1), d'une valeur d'intensité lumineuse de chambre d'analyse pour ledit instant de mesure,
    le procédé étant **caractérisé en ce qu'**il comprend en outre, pour une pluralité d'instants de mesure :

    - une détermination (S03) à partir de l'image du support d'analyse (1), de valeurs d'intensité lumineuse de chambres de référence (6) pour ledit instant de mesure,
- une détermination (S04), à partir des valeurs d'intensité lumineuse de chambres de référence (6) pour ledit instant de mesure, d'une relation d'étalonnage liant la valeur d'intensité lumineuse et concentration d'endotoxines à cet instant de mesure ;

le procédé comprenant également, pour une pluralité d'instants de mesure:

- une détermination (S05) d'au moins une mesure de concentration d'endotoxines dans l'échantillon biologique à cet instant de mesure à partir d'une relation d'étalonnage pour ledit instant de mesure et de la valeur d'intensité de chambre d'analyse audit instant de mesure ; et
- une détermination (S06) d'une évolution temporelle de la mesure de concentration d'endotoxines dans l'échantillon biologique au cours de la durée de mesure à partir de mesures de concentration d'endotoxines pour plusieurs instants de mesure.

**2.** Procédé selon la revendication 1, comprenant ensuite une mise en œuvre d'une action fonction de l'évolution temporelle de la mesure de concentration d'endotoxines (S07).

**3.** Procédé selon la revendication 2, dans lequel, l'action mise en œuvre comprend l'arrêt du procédé ou une alerte en fonction d'une stabilité de ladite évolution temporelle de la mesure de concentration d'endotoxines ou d'une décroissance de la mesure de concentration d'endotoxines.

**4.** Procédé selon l'une quelconque des revendications précédentes, dans lequel une relation d'étalonnage est déterminée pour chaque instant de mesure de la durée de mesure.

**5.** Procédé selon l'une quelconque des revendications 1 à 3, dans lequel la relation d'étalonnage pour un instant de mesure est une relation d'étalonnage déterminée à partir des valeurs d'intensité lumineuse des chambres de référence d'un instant de mesure précédent.

**6.** Procédé selon l'une quelconque des revendications précédentes, dans lequel l'évolution temporelle d'une mesure de concentration d'endotoxines dans l'échantillon biologique est déterminée plusieurs fois au cours de la durée de mesure, à chaque fois à la suite d'un instant de mesure pris en compte dans ladite évolution temporelle.

**7.** Procédé selon l'une quelconque des revendications précédentes, des réactifs d'analyse présents dans des chambres d'analyse (2) et des réactifs de référence présents dans des chambres de référence (6) comprennent un facteur C recombinant et un substrat fluorigène des chambres de référence (6) comprenant des endotoxines à des concentrations prédéterminées.

**8.** Procédé selon la revendication précédente, dans lequel au moins une chambre de référence (6a) est dépourvue d'endotoxine.

**9.** Procédé selon l'une quelconque des revendications précédentes, dans lequel le support d'analyse (1) comprend une pluralité de chambres d'analyse (2), et la détermination la détermination (S03) d'une valeur d'intensité lumineuse de chambre d'analyse pour ledit instant de mesure comprend la détermination d'une valeur statistiquement représentative de valeurs d'intensité lumineuse de plusieurs desdites chambres d'analyse (2).

**10.** Instrument d'analyse (10) comprenant un imageur (12) définissant un champ de vue (11), l'instrument d'analyse étant configuré pour recevoir un support d'analyse (1) dans le champ de vue (11) de l'imageur (12), ledit support d'analyse (1) comprenant au moins une chambre d'analyse (2) configurée pour recevoir un échantillon biologique, et une pluralité de chambres de référence (6) configurées pour recevoir un liquide de référence, les chambres de référence (6) étant pourvues de réactifs de référence et de concentrations de référence, les réactifs de référence étant aptes à causer une réaction de luminescence en présence du liquide de référence en fonction de la concentration d'endotoxines dans la chambre de référence (6) correspondante, la chambre d'analyse (2) étant pourvue de réactifs d'analyse aptes à causer une réaction de luminescence en présence d'endotoxines de l'échantillon biologique, le système étant configuré pour mettre en œuvre au moins les étapes du procédé selon l'une quelconque des revendications précédentes.

**Patentansprüche**

**1.** Verfahren zur Quantifizierung von Endotoxinen einer biologischen Probe durch ein Analysegerät (10), umfassend eine Abbildungsvorrichtung (12), die ein Blickfeld (11) definiert, einen Analyseträger (1), der in das Blickfeld (11) des Analysegeräts (10) eingeführt wird, wobei der Analyseträger (1) wenigstens eine Analysekammer (2), konfiguriert zum Aufnehmen der biologischen Probe, und eine Vielzahl von Referenzkammern (6), konfiguriert zum Aufnehmen einer Referenzflüssigkeit, umfasst, wobei das Verfahren zunächst ein Platzieren (S01) der biologischen Probe in der Analysekammer (2) und der Referenzflüssigkeit in der Vielzahl von Referenzkammern (6) umfasst, wobei die Referenzkammern (6) mit Referenzreagenzien und verschiedenen Konzentrationen von Endotoxinen versehen sind, wobei die Referenzreagenzien zum Verursachen einer Lumineszenzreaktion bei Anwesenheit der Referenzflüssigkeit gemäß der Konzentration von Endotoxinen in der entsprechenden Referenzkammer (6) geeignet sind, wobei die Analysekammer (2) mit Analysereagenzien, die zum Verursachen einer Lumineszenzreaktion bei Anwesenheit von Endotoxinen der biologischen Probe geeignet sind, versehen ist,

wobei das Verfahren, für jeden Messzeitpunkt eines Messzeitraums, die Erfassung (S02) eines Bilds des Analyseträgers (1) an besagtem Messzeitpunkt und die Bestimmung (S03) eines Analysekammer-Lichtintensitätswerts für den besagten Messzeitpunkt aus dem Bild des Analyseträgers (1) umfasst,
wobei das Verfahren weiterhin, für eine Vielzahl von Messzeitpunkten,
**dadurch gekennzeichnet, dass** das Verfahren umfasst:

- eine Bestimmung (S03) von Lichtintensitätswerten der Referenzkammern (6) für die besagten Messzeitpunkte aus dem Bild des Analyseträgers (1),
- eine Bestimmung (S04), ausgehend von den Lichtintensitätswerten der Referenzkammern (6) für den besagten Messzeitpunkt, einer Kalibrierungsbeziehung, welche den Lichtintensitätswert mit der Konzentration von Endotoxinen an diesem Messzeitpunkt in Verbindung bringt;

wobei das Verfahren, für eine Vielzahl von Messzeitpunkten, ferner umfasst:

- eine Bestimmung (S05) von wenigstens einem Messwert der Konzentration von Endotoxinen in der biologischen Probe an diesem Messzeitpunkt aus einer Kalibrierungsbeziehung für den besagten Messzeitpunkt und dem Analysekammer-Intensitätswert an besagtem Messzeitpunkt; und
- eine Bestimmung (S06) einer zeitlichen Entwicklung des Messwerts der Konzentration von Endotoxinen in der biologischen Probe im Verlauf des Messzeitraums aus Messwerten der Konzentration von Endotoxinen für mehrere Messzeitpunkte.

2. Verfahren nach Anspruch 1, das anschließend die Durchführung einer Maßnahme abhängig von der zeitlichen Entwicklung des Messwerts der Konzentration von Endotoxinen (S07) umfasst.

3. Verfahren nach Anspruch 2, wobei die durchgeführte Maßnahme die Unterbrechung des Verfahrens oder eine Warnmeldung in Abhängigkeit von einer Stabilität der zeitlichen Entwicklung des Messwerts der Konzentration von Endotoxinen oder einer Abnahme des Messwerts der Konzentration von Endotoxinen umfasst.

4. Verfahren nach einem der vorhergehenden Ansprüche, wobei eine Kalibrierungsbeziehung für jeden Messzeitpunkt des Messzeitraums bestimmt wird.

5. Verfahren nach einem der Ansprüche 1 bis 3, wobei die Kalibrierungsbeziehung für einen Messzeitpunkt eine Kalibrierungsbeziehung ist, die aus den Lichtintensitätswerten der Referenzkammern eines vorhergehenden Messzeitpunkts bestimmt wird.

6. Verfahren nach einem der vorhergehenden Ansprüche, wobei die zeitliche Entwicklung eines Messwerts der Konzentration von Endotoxinen in der biologischen Probe mehrere Male im Verlauf des Messzeitraums bestimmt wird, und zwar jedes Mal im Anschluss daran, dass ein Messzeitpunkt in die besagte zeitliche Entwicklung einbezogen wurde.

7. Verfahren nach einem der vorhergehenden Ansprüche, wobei Analysereagenzien, die in Analysekammern (2) vorliegen, und Referenzreagenzien, die in Referenzkammern (6) vorliegen, einen rekombinanten Faktor C und ein fluorogenes Substrat der Referenzkammern (6), das Endotoxine bei vorbestimmten Konzentrationen umfasst, umfassen.

8. Verfahren nach dem vorhergehenden Anspruch, wobei wenigstens eine Referenzkammer (6a) frei von Endotoxin ist.

9. Verfahren nach einem der vorhergehenden Ansprüche, wobei der Analyseträger (1) eine Vielzahl von Analysekammern (2) umfasst, und die Bestimmung (S03) eines Analysekammer-Lichtintensitätswerts für den besagten Messzeitpunkt die Bestimmung eines statistisch repräsentativen Werts der Lichtintensitätswerte von mehreren der besagten Analysekammern (2) umfasst.

10. Analysegerät (10), umfassend eine Abbildungsvorrichtung (12), die ein Blickfeld (11) definiert, wobei das Analysegerät zum Aufnehmen eines Analyseträgers (1) im Blickfeld (11) der Abbildungsvorrichtung (12) konfiguriert ist, wobei der Analyseträger (1) wenigstens eine Analysekammer (2), konfiguriert zum Aufnehmen einer biologischen Probe, und eine Vielzahl von Referenzkammern (6), konfiguriert zum Aufnehmen einer Referenzflüssigkeit, umfasst, wobei die Referenzkammern (6) mit Referenzreagenzien und Referenzkonzentrationen versehen sind, wobei die Referenzreagenzien zum Verursachen einer Lumineszenzreaktion bei Anwesenheit der Referenzflüssigkeit gemäß der Konzentration von Endotoxinen in der entsprechenden Referenzkammer (6) geeignet sind, wobei die Analysekammer (2) mit Analysereagenzien, die zum Verursachen einer Lumineszenzreaktion bei Anwesenheit von Endotoxinen der biologischen Probe geeignet sind, versehen ist, wobei das System zum Durchführen wenigstens der Schritte des Verfahrens nach einem der vorhergehenden Ansprüche konfiguriert ist.

**Claims**

1. Process for quantifying endotoxins in a biological sample via an analytical instrument (10) comprising an imager (12) defining a field of view (11), an analytical support (1) being introduced into the field of view (11) of the analytical instrument (10), said analytical support (1) comprising at least one analysis chamber (2) configured to receive said biological sample and a plurality of reference chambers (6) configured to receive a reference liquid, the process first involving placing (S01) the biological sample in said analysis chamber (2) and reference liquid in the plurality of reference chambers (6), reference chambers (6) being provided with reference reagents and different concentrations of endotoxins, said reference reagents being capable of causing a luminescence reaction in the presence of the reference liquid as a function of the endotoxin concentration in the corresponding reference chamber (6), the analysis chamber (2) being provided with analytical reagents that are capable of causing a luminescence reaction in the presence of endotoxins of the biological sample,

   the process comprising, for each measurement instant of a measurement period, the acquisition (S02) at said measurement instant of an image of the analytical support (1) and the determination (S03), from said image of the analytical support (1), of an analysis chamber light intensity value for said measurement instant, the process also comprising, for a plurality of measurement instants, **characterized in that** the process comprises:

   - the determination (S03), from the image of the analytical support (1), of light intensity values of reference chambers (6) for said measurement instant,
   - the determination (S04), from the light intensity values of reference chambers (6) for said measurement instant, of a calibration relationship linking the light intensity value and the endotoxin concentration at said measurement instant;

   the process also comprising, for a plurality of measurement instants:

   - the determination (S05) of at least one measurement of endotoxin concentration in the biological sample at said measurement instant from a calibration relationship for said measurement instant and the analysis chamber intensity value at said measurement instant; and
   - the determination (S06) of a temporal evolution of the endotoxin concentration measurement in the biological sample over the measurement period from endotoxin concentration measurements for several measurement instants.

2. Process according to Claim 1, subsequently comprising the implementation of an action as a function of the temporal evolution of the endotoxin concentration measurement (S07).

3. Process according to Claim 2, in which the action performed involves stopping the process or issuing an alert as a function of the stability of said temporal evolution of the endotoxin concentration measurement or of a decrease in the endotoxin concentration measurement.

4. Process according to any one of the preceding claims, in which a calibration relationship is determined for each measurement instant of the measurement period.

5. Process according to any one of Claims 1 to 3, in which the calibration relationship for a measurement instant is a calibration relationship determined from the light intensity values of the reference chambers of a preceding measurement instant.

6. Process according to any one of the preceding claims, in which the temporal evolution of a measurement of endotoxin concentration in the biological sample is determined several times during the measurement period, each time following a measurement instant taken into account in said temporal evolution.

7. Process according to any one of the preceding claims, analytical reagents present in analysis chambers (2) and reference reagents present in reference chambers (6) comprising a recombinant factor C and a fluorogenic substrate of the reference chambers (6) comprising endotoxins at predetermined concentrations.

8.  Process according to the preceding claim, in which at least one reference chamber (6a) is free of endotoxin.

9.  Process according to any one of the preceding claims, in which the analytical support (1) comprises a plurality of analysis chambers (2), and the determination (S03) of an analysis chamber light intensity value for said measurement instant comprises the determination of a statistically representative value of light intensity values of a plurality of said analysis chambers (2).

10. Analytical instrument (10) comprising an imager (12) defining a field of view (11), the analytical instrument being configured to receive an analytical support (1) in the field of view (11) of the imager (12), said analytical support (1) comprising at least one analysis chamber (2) configured to receive a biological sample, and a plurality of reference chambers (6) configured to receive a reference liquid, the reference chambers (6) being provided with reference reagents and reference concentrations, the reference reagents being capable of causing a luminescence reaction in the presence of the reference liquid as a function of the endotoxin concentration in the corresponding reference chamber (6), the analysis chamber (2) being provided with analytical reagents that are capable of causing a luminescence reaction in the presence of endotoxins of the biological sample, the system being configured to perform at least the steps of the process according to any one of the preceding claims.

FIG 1

FIG 2

# FIG 3

S01 — Mise en place

S02 — acquisition d'une image

S03 — Extraction des valeurs d'intensité lumineuses

S04 — Détermination d'une relation

S05 — Détermination d'une concentration en endotoxines

S06 — Détermination de l'évolution temporelle d'une concentration d'endotoxines

S07 — Mise en œuvre d'une action fonction de l'évolution temporelle

## FIG 4

# FIG 5

# FIG 6

$y = 1{,}0176x + 7{,}5409$
$R^2 = 0{,}9998$

# FIG 7

# FIG 8

**EP 4 374 158 B1**

## RÉFÉRENCES CITÉES DANS LA DESCRIPTION

**Documents brevets cités dans la description**

- WO 2020185646 A1